# EUROPÄISCHE PATENTSCHRIFT

(11) **EP 1 747 185 B1**
(45) Veröffentlichungstag und Bekanntmachung des Hinweises auf die Patenterteilung: **04.03.2015**
(21) Anmeldenummer: 05741200.9
(22) Anmeldetag: 30.04.2005
(51) Int. Cl.: C07C 68/02, C07C 69/96

(54) **VERFAHREN ZUR HERSTELLUNG VON DIMETHYLDICARBONATEN**
METHOD FOR PRODUCING DIMETHYL DICARBONATES
PROCEDE POUR LA PRODUCTION DE BICARBONATES DE DIMETHYLE

(30) Priorität: 13.05.2004 DE 102004023607
(43) Veröffentlichungstag der Anmeldung: 31.01.2007
(73) Patentinhaber: LANXESS Deutschland GmbH, 50569 Köln (DE)
(72) Erfinder: PRINZ, Thomas, 51371 Leverkusen (DE); ECKERT, Markus, 50931 Köln (DE); BUSCHHAUS, Hans-Ulrich, 47800 Krefeld (DE); KAHLERT, Steffen, 42799 Leichlingen (DE)
(86) Internationale Anmeldenummer: PCT/EP2005/004724
(87) Internationale Veröffentlichungsnummer: WO 2005/110964

(56) Entgegenhaltungen:
- DE-A1- 1 418 849
- DE-B- 1 181 195
- FR-A- 1 483 560

## Beschreibung

Die vorliegende Erfindung betrifft ein neues Verfahren zur Herstellung von Dimethyldicarbönat aus Halogenameisensäuremethylestern unter Verwendung spezieller tertiärer Alkylamine als Katalysatoren.

Dialkyldicarbonate finden z.B. als Katalysatoren zur Oxidation von sterisch anspruchsvollen Aminen, als Bestandteile von Elektrolytflüssigkeiten oder als Bestandteile von antimikrobiellen Reagenzien Verwendung. Dialkyldicarbonate werden in der Literatur auch als Dialkyl-pyrocarbonate bezeichnet.

Aus DAS 1 210 853 (1961) ist bekannt, Kohlensäure- oder Carbonsäurehalogenide mit organischen Hydroxylverbindungen oder deren Alkali- oder Erdalkalisalzen und organischen, mit Wasser nicht mischbaren Lösungsmitteln und mindestens äquivalenten Mengen an Alkali- oder Erdalkalihydroxiden oder -carbonaten in einem zweiphasigen System, sowie unter Verwendung katalytischer Mengen an tertiären Aminen oder ihren Quaternierungsprodukten, umzusetzen, wobei als Amine oder deren Quaternierungsprodukte solche eingesetzt werden, die mindestens eine an Stickstoff gebundene ω-Hydroxyalkyl-, ω-Hydroxyalkyläther- oder ω-Hydroxyalkylpolyäthergruppe tragen.

In DE-A 1 418 849 (1961) werden weiterhin tertiäre Amine als besonders geeignete Katalysatoren für die Herstellung von Säurederivaten beschrieben, deren tertiären Stickstoffatome sterisch nicht gehindert sind, ausgenommen tertiäre Amine, die als Substituenten am Stickstoff ebensolche ω-Hydroxyalkyl-, ω-Hydroxyalkyläther- oder ω-Hydroxyalkylpolyäthergruppe tragen. Neben Triethylamin und Tri-n-butylamin kommen hier deshalb Amine zum Einsatz, die mindestens eine Methylgruppe am Stickstoff tragen, wie z.B. N-Methyl-di-n-stearylamin. Diese Katalysatoren besitzen aber u.a. den Nachteil, dass sie nicht nur die Bildung sondern auch die Zersetzung des Produktes katalysieren, was zu einer Verringerung der Ausbeute führt.

Weitere Verfahren zur Herstellung von Dialkyldicarbonaten aus Carbonsäurehalogeniden mit tertiären Aminen, bei denen das Stickstoffatom nicht sterisch gehindert ist, sind aus der DE-A 1181195 und der FR-A 1483 560 bekannt.

Es bestand daher Bedarf an einem verbesserten Herstellungsverfahren, welches das Zielprodukt in höherer Ausbeute liefert.

Überraschenderweise wurde gefunden, dass Dimethyldicarbonat aus Halogenameisensäuremethylester durch Umsetzung mit Alkali- oder Erdalkalihydroxiden oder - carbonaten besonders vorteilhaft erhalten werden können, wenn man als Katalysator spezielle langkettige tertiäre Alkylamine einsetzt. Diese zeichnen sich durch eine hohe katalytische Aktivität aus, ohne zersetzend auf das Endprodukt zu wirken und können leicht vom Produkt, z.B. destillativ, abgetrennt werden.

Gegenstand der vorliegenden Erfindung ist demnach ein Verfahren zur Herstellung von Dimethyldicarbonät durch Umsetzung der entsprechenden Halogenameisensäurmethylester mit Alkali- oder Erdalkalihydroxiden und/oder -carbonaten in Gegenwart von mit Wasser nicht mischbaren organischen Lösungsmitteln und in Gegenwart eines Katalysators dadurch gekennzeichnet ist, dass als Katalysator mindestens ein tertiäres Alkylamin der Formel (I)

NR¹R²R³ (I)

worin
R¹, R² und R³ unabhängig voneinander für geradkettiges oder verzweigtes C₆-C₂₅-Alkyl steht,
eingesetzt wird.

Bevorzugt werden bei der Durchführung des erfindungsgemäßen Verfahrens als Katalysator tertiäre Alkylamine der Formel (I) eingesetzt, worin R¹, R² und R³ unabhängig voneinander für geradkettiges oder verzweigtes C₈-C₁₆-Alkyl stehen.

Bei der Durchführung des erfindungsgemäßen Verfahrens besonders bevorzugte Katalysatoren sind Trisoctylamin, Trisisooctylamin, Trislaurylamin, Triscaprylylamin oder beliebige Mischungen dieser Amine.

Das erfindungsgemäße Verfahren dient zur Herstellung von Dimethyldicarbonat durch Umsetzung von Halogenameisensäuremethylester der Formel (II) 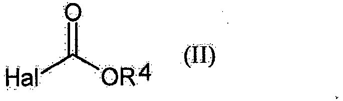 worin
- Hal: für Halogen, bevorzugt für F, Cl, Br, I, insbesondere für Chlor steht, und
- R⁴: für Methylsteht,
dadurch gekennzeichnet, dass die Umsetzung in Gegenwart von mindestens einem tertiären Alkylamin der Formel (I)

NR¹R²R³

worin
- R¹, R² und R³: unabhängig voneinander für geradkettiges oder verzweigtes C₆-C₂₅-Alkyl, vorzugsweise für geradkettiges oder verzweigtes C₈-C₁₆-Alkyl, stehen,
als Katalysator durchgeführt wird.

Als Alkali- oder Erdalkalihydroxide oder-carbonate kommen beispielsweise LiOH, NaOH, KOH, Li₂CO₃, Na₂CO₃, K₂CO₃ in Frage. Vorzugsweise werden Alkalihydroxide verwendet, wie Natrium- und Kaliumhydroxid, die vorzugsweise in Form von wässrigen Lösungen zum Einsatz gelangen. Beispielsweise kann man 1 bis 50 gew.-%ige wässrige Alkalihydroxidlösungen verwenden. Bevorzugt sind 5 bis 35 gew.-%ige Lösungen, besonders bevorzugt 10 bis 25 gew.%ige Lösungen. Die Alkali- oder Erdalkalihydroxide oder-carbonate kann man beispielsweise in Mengen von 80 bis 120 Mol-% bezogen auf eingesetzten Halogenameisensäureester einsetzen. Vorzugsweise liegt diese Menge im Bereich von 90 bis 110 Mol-%, besonders bevorzugt im Bereich von 95 bis 105 Mol-%.

Als mit Wasser nicht mischbare organische Lösungsmittel kommen beispielsweise aliphatische und aromatische Kohlenwasserstoffe, halogenierte Kohlenwasserstoffe, mit Wasser nicht mischbare Ether oder Ester sowie Dialkylcarbonate in Frage. Bevorzugt sind Cyclohexan, Toluol, Xylol, Methylenchlorid und Diethylether, insbesondere Toluol und Methylenchlorid.

Das mit Wasser nicht mischbare organische Lösungsmittel kann man beispielsweise in Mengen von 20 bis 90 Gew.-%, bevorzugt von 30 bis 80 Gew.-%, besonders bevorzugt von 40 bis 70 Gew.-% bezogen auf den Halogenameisensäureester der Formel (I) einsetzen.

Der Katalysator der Formel (I) wird im Allgemeinen bezogen auf Halogenameisensäureester, in einer Menge von 0,001 bis 0,5, vorzugsweise von 0,005 bis 0,05 Mol-% eingesetzt.

Das erfindungsgemäße Verfahren kann in einem Druckbereich von 1 bis 10 bar, vorzugsweise von 1 bis 1,5 bar durchgeführt werden.

Die Reaktionstemperatur kann beispielsweise zwischen -10°C und der Siedetemperatur (bei Normaldruck) des eingesetzten Halogenameisensäureesters liegen. Vorzugsweise liegt sie im Bereich 0 bis 50°C.

Es ist vorteilhaft, während der Durchführung des erfindungsgemäßen Verfahrens für eine gute Durchmischung zu sorgen, z.B. durch Einsatz von Rührwerken, Strömungsstörern oder Umwälzpumpen.

Das erfindungsgemäße Verfahren kann sowohl batchweise als auch kontinuierlich durchgeführt werden. Bei batchweiser Arbeitsweise wird die Umsetzung bevorzugt in einem Rührkessel durchgeführt. Die Reaktion ist hierbei je nach Größe des Ansatzes und der vorhandenen Kühlleistung im allgemeinen nach 10 Minuten bis 3 Stunden beendet.

Vorzugsweise wird das erfindungsgemäße Verfahren kontinuierlich unter Verwendung eines Rührkessels, einer Rührkesselkaskade oder eines Rohrreaktors durchgeführt. Hierbei beträgt die mittlere Verweilzeit im Reaktor in der Regel zwischen 1 und 60 Minuten, bevorzugt zwischen 6 und 45 Minuten und besonders bevorzugt zwischen 10 und 20 Minuten.

Nach der Durchführung des erfindungsgemäßen Verfahrens, gegebenenfalls nach Abkühlung trennt sich das Reaktionsgemisch in zwei Phasen auf. Die organische Phase enthält neben dem Lösungsmittel das hergestellte Dimethyldicarbonat und gegebenenfalls geringe Mengen unumgesetzten Halogenameisensäureester sowie den Katalysator. Die wässrige Phase enthält neben Wasser die gebildeten anorganischen Salze.

Aus der organischen Phase kann man das Reaktionsprodukt durch mehrstufige Destillation in hoher Reinheit gewinnen. Der Katalysator kann dabei als Schwersieder abgetrennt werden und gegebenenfalls nach einer Aufreinigung wieder in dem erfindungsgemäßen Verfahren als Katalysator eingesetzt werden (Recyclisierung).

Es ist ein besonderer und überraschender Vorteil des erfindungsgemäßen Verfahrens, dass der Katalysator praktisch nicht die Zersetzung der Dimethyldicarbonat nach der Reaktion katalysiert und daher durch Destillation abgetrennt werden kann, wodurch die isolierte Ausbeute an Endprodukt im Vergleich zu herkömmlichen Verfahren höher ist.

### Beispiele

### Beispiel 1

1,17 g Trilaurylamin wurde in einer Mischung aus 50 ml Toluol und 25 ml Wasser vorgelegt und 43,8 g Chlorameisensäuremethylester bei einer Innentemperatur von 5°C unter Rühren zugetropft. Anschließend wurde eine Lösung von 35,6 g einer 45 %igen Natronlauge in 96,5 ml Wasser innerhalb von 30 Minuten zugetropft und die Temperatur bei 15°C gehalten. Nachdem 40 Minuten nachgerührt wurde, wurden die Phasen getrennt. In der organischen Phase befanden sich 23,1 g Dimethyldicarbonat (85 % d.Th.).

### Vergleichsbeispiel 2

Zu einer Mischung aus 138 g Chlorameisensäureisobutylester, 138 g Toluol und 1,42 g Triisooctylamin wurde innerhalb von 35 Minuten eine Mischung aus 290 g 13,8 gew.-%iger wässriger Natronlauge getropft. Die Reaktionstemperatur wurde dabei bei 30°C gehalten. Es wurden 15 Minuten nachgerührt, abgekühlt und abschließend die Phasen getrennt. In der organischen Phase befanden sich 98,5 g Diisobutyldicarbonat (= 90 % d.Th.).

### Beispiel 3

Zu einer Mischung aus 1.15 g Triisooctylamin, 180 ml Toluol und 156,9 g Chlorameisensäuremethylester wurden 473 g 12,1 %-ige Natronlauge binnen 45 Minuten so zugepumpt, dass unter Kühlung die Temperatur bei ca. 17°C gehalten werden konnte. Es wurde 30 Minuten nachgerührt und anschließend die Phasen getrennt. In der organischen Phase befanden sich 86,7 g Dimethyldicarbonat (91 % d.Th.) und 5,3 g Chlorameisensäuremethylester.

## Patentansprüche

1. Verfahren zur Herstellung von Dimethyldicarbonat durch Umsetzung der Halogenameisensäuremethylester mit mindestens einem Alkalihydroxid, Erdalkalihydroxid und/oder -carbonat in Gegenwart von mindestens einem, mit Wasser nicht mischbaren organischen Lösungsmittel und in Gegenwart eines Katalysators **dadurch gekennzeichnet, dass** als Katalysator mindestens ein tertiäres Alkylamin der Formel (I)
NR¹R²R³ (I)
worin
R¹, R² und R³ unabhängig voneinander für geradkettiges oder verzweigtes C₆-C₂₅-Alkyl stehen,
eingesetzt wird.

2. Verfahren gemäß Anspruch 1, daduch gekennzeichnet dass als Katalysator mindestens ein tertiäres Alkylamin der Formel (I) gemäß Anspruch 1 eingesetzt wird, worin R¹, R² und R³ unabhängig voneinander für geradkettiges oder verzweigtes C₈-C₁₆-Alkyl stehen.

3. Verfahren gemäß wenigstens einem der Ansprüche 1 und 2, **dadurch gekennzeichnet, dass** als Katalysator mindestens ein Amin aus der Reihe Trisoctylamin, Trisisooctylamin, Trislaurylamin, Triscaprylylamin eingesetzt wird.

4. Verfahren gemäß wenigstens einem der Ansprüche 1 bis 3, **dadurch gekennzeichnet, dass** die Alkalihydroxide, Erdalkalihydroxide und/oder-carbonate in Form wässriger Lösungen einsetzt werden.

5. Verfahren gemäß wenigstens einem der Ansprüchen 1 bis 4, **dadurch gekennzeichnet, dass** man mindestens ein mit Wasser nicht mischbares organisches Lösungsmittel aus der Reihe der aliphatischen und aromatischen Kohlenwasserstoffe, der chlorierten Kohlenwasserstoffe, der Dialkylcarbonate oder der mit Wasser nicht mischbaren Ether und Ester einsetzt.

6. Verfahren gemäß wenigstens einem der Ansprüche 1 bis 5, **dadurch gekennzeichnet, dass** die Menge an eingesetztem tertiären Trialkylamin der Formel (I) 0,001 bis 0,5 Mol-%, bezogen auf Halogenameisensäureester, beträgt.

7. Verfahren gemäß wenigstens einem der Ansprüche 1 bis 6, **dadurch gekennzeichnet, dass** die Reaktion bei einer Temperatur zwischen -10°C und der Siedetemperatur bei Normaldruck des eingesetzten Halogenameisensäureesters durchgeführt wird.

8. Verfahren gemäß wenigstens einem der Ansprüche 1 bis 7, **dadurch gekennzeichnet, dass** die Umsetzung in kontinuierlicher Arbeitsweise durchgeführt wird.

9. Verfahren gemäß wenigstens einem der Ansprüche 1 bis 8, **dadurch gekennzeichnet, dass** nach Beendigung der Umsetzung die Aufarbeitung des Reaktionsgemisches zur Abtrennung des Dimethyldicarbonats durch Phasentrennung und anschließender mehrstufiger Destillation der organischen Phase erfolgt.

## Claims

1. Process for preparing dimethyl dicarbonate by reacting the methyl haloformates with at least one alkali metal hydroxide, alkaline earth metal hydroxide and/or carbonate in the presence of at least one water-immiscible organic solvent and in the presence of a catalyst, **characterized in that** the catalyst used is at least one tertiary alkylamine of the formula (I)
NR¹R²R³ (I)
where
R¹, R² and R³ are each independently straight-chain or branched C₆-C₂₅-alkyl.

2. Process according to Claim 1, **characterized in that** the catalyst used is at least one tertiary alkylamine of the formula (I) according to Claim 1 where R¹, R² and R³ are each independently straight-chain or branched C₈-C₁₆-alkyl.

3. Process according to at least one of Claims 1 and 2, **characterized in that** the catalyst used is at least one amine from the group of trisoctylamine, trisisooctylamine, trislauryl-amine, triscaprylylamine.

4. Process according to at least one of Claims 1 to 3, **characterized in that** the alkali metal hydroxides, alkaline earth metal hydroxides and/or carbonates are used in the form of aqueous solutions.

5. Process according to at least one of Claims 1 to 4, **characterized in that** at least one water-immiscible organic solvent from the group of the aliphatic and aromatic hydrocarbons, the chlorinated hydrocarbons, the dialkyl carbonates or the water-immiscible ethers and esters is used.

6. Process according to at least one of Claims 1 to 5, **characterized in that** the amount of tertiary trialkylamine of the formula (I) used is 0.001 to 0.5 mol% based on the haloformic esters.

7. Process according to at least one of Claims 1 to 6, **characterized in that** the reaction is carried out at a temperature between -10°C and a boiling point at standard pressure of the haloformic ester used.

8. Process according to at least one of Claims 1 to 7, **characterized in that** the reaction is carried out in continuous mode.

9. Process according to at least one of Claims 1 to 8, **characterized in that**, on completion of the reaction, the reaction mixture is worked up to remove the dimethyl dicarbonate by phase separation and subsequent multistage distillation of the organic phase.

## Revendications

1. Procédé pour la préparation de dicarbonate de diméthyle par transformation de l'ester méthylique de l'acide halogénoformique avec au moins un hydroxyde de métal alcalin, un hydroxyde et/ou un carbonate de métal alcalino-terreux en présence d'au moins un solvant organique non miscible à l'eau et en présence d'un catalyseur, **caractérisé en ce qu'**on utilise comme catalyseur au moins une alkylamine tertiaire de formule (I)
NR¹R²R³ (I)
dans laquelle
R¹, R² et R³ représentent, indépendamment les uns des autres, C₆-C₂₅-alkyle linéaire ou ramifié.

2. Procédé selon la revendication 1, **caractérisé en ce qu'**on utilise comme catalyseur au moins une alkylamine tertiaire de formule (I) selon la revendication 1, dans laquelle R¹, R² et R³ représentent, indépendamment les uns des autres, C₈-C₁₆-alkyle linéaire ou ramifié.

3. Procédé selon au moins l'une quelconque des revendications 1 et 2, **caractérisé en ce qu'**on utilise comme catalyseur au moins une amine de la série trioctylamine, triiso-octylamine, trilaurylamine, tricaprylylamine.

4. Procédé selon au moins l'une quelconque des revendications 1 à 3, **caractérisé en ce que** les hydroxydes de métal alcalin, les hydroxydes et/ou carbonates de métal alcalino-terreux sont utilisés sous forme de solution aqueuse.

5. Procédé selon au moins l'une quelconque des revendications 1 à 4, **caractérisé en ce qu'**on utilise au moins un solvant organique non miscible à l'eau de la série des hydrocarbures aliphatiques et aromatiques, des hydrocarbures chlorés, des carbonates de dialkyle ou des éthers et esters non miscibles à l'eau.

6. Procédé selon au moins l'une quelconque des revendications 1 à 5, **caractérisé en ce que** la quantité de trialkylamine tertiaire utilisée de formule (I) est de 0,001 à 0,5% en mole, par rapport à l'ester de l'acide halogénoformique.

7. Procédé selon au moins l'une quelconque des revendications 1 à 6, **caractérisé en ce que** la réaction est réalisée à une température entre -10°C et la température d'ébullition à pression normale de l'ester de l'acide halogénoformique utilisé.

8. Procédé selon au moins l'une quelconque des revendications 1 à 7, **caractérisé en ce que** la transformation est réalisée dans un mode de travail continu.

9. Procédé selon au moins l'une quelconque des revendications 1 à 8, **caractérisé en ce qu'**après la fin de la transformation, on réalise le traitement du mélange réactionnel pour séparer le dicarbonate de diméthyle par séparation des phases et distillation consécutive en plusieurs étapes de la phase organique.
